(19) Europäisches Patentamt
European Patent Office
Office européen des brevets

(11) Veröffentlichungsnummer: **0 480 482 A2**

(12) # EUROPÄISCHE PATENTANMELDUNG

(21) Anmeldenummer: **91121666.1**

(22) Anmeldetag: **19.09.87**

(51) Int. Cl.5: **C07C 45/58**, C07C 47/228, C07C 47/02

Diese Anmeldung is am 18 - 12 - 1991 als Teilanmeldung zu der unter INID-Kode 60 erwähnten Anmeldung eingereicht worden.

(30) Priorität: **25.09.86 DE 3632529**

(43) Veröffentlichungstag der Anmeldung: **15.04.92 Patentblatt 92/16**

(60) Veröffentlichungsnummer der früheren Anmeldung nach Art. 76 EPÜ: **0 262 532**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms 1(DE)**
Erfinder: **Hupfer, Leopold, Dr.**
**Waltershoehe 3**
**W-6701 Friedelsheim(DE)**
Erfinder: **Lermer, Helmut, Dr.**
**D 3,4**
**W-6800 Mannheim 1(DE)**

(54) **Verfahren zur Herstellung von Aldehyden und/oder Ketonen durch Umsetzung von Epoxiden.**

(57) Die Anmeldung betrifft ein Verfahren zur Herstellung von Aldehyden und Ketonen aus Epoxiden der Formel

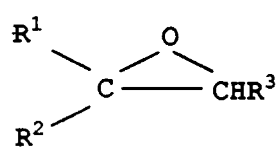

in der $R^1$ und $R^2$ Alkyl-, Alkenyl-, Aryl-, Alkoxy- oder Aralkylreste und $R^3$ diese oder Wasserstoff bedeuten, durch katalytische Umlagerung in Gegenwart verschiedener Katalysatoren, insbesondere hydrothermal hergestellten Phosphaten, Phosphaten des B, Fe, Zr, Ce, $SiO_2$, Borsäure auf Basis $SiO_2$ bzw. $Al_2O_3$.

EP 0 480 482 A2

Die Erfindung betrifft ein Verfahren zur Herstellung von Aldehyden und/oder Ketonen durch Umsetzung von Epoxiden an hydrothermal hergestellten Phosphaten, an Phosphaten von B, Fe, Ce, Zr bzw. Borsäure auf Katalysatorträgern oder an Siliciumdioxid.

Verfahren zur Herstellung von Aldehyden und Ketonen sind in der Literatur mehrfach beschrieben. So sind verschiedene Verfahren bekannt, bei denen saure Homogenkatalysatoren wie Phosphorsäure, Bortrifluorid-Diethyletherat, Zinn-IV-Chlorid und Palladiumkomplexe für die Flüssigphasenreaktion verwendet werden. Für die Gasphasenreaktionen werden Aluminiumoxide und Aluminiumsilikate als Katalysatoren für die Umlagerung genannt.

Es ist auch bekannt, Butylenoxid an dotierten A-Zeolithen zu einem Gemisch aus Butyraldehyd, cis-trans-But-2-en-ol, Butanol und Methylethylketon umzusetzen. Doch ist die Selektivität bei dieser Reaktion noch ungenügend. Auch ist der A-Zeolith-Katalysator nach seiner Desaktivierung durch Koks nicht zu regenerieren, da bei den hierfür notwendigen Temperaturen von etwa 500 °C die Kristallstruktur des Zeolith zerstört wird. Dies gilt auch für die Herstellung von 2-(4')-Isobutyl-phenyl-propanalaus dem 2-4'-Isobutyl-phenyl-2-methyloxiran an s Å Molekularsieben (JP 3031-637).

Für die Umsetzung von Propylenoxid zu Aceton oder Propionaldehyd an alkalidotierten X-Zeolithen ist es notwendig in Abwesenheit stark acider Zentren zu arbeiten.

Cyclododecanon hat man an Pd- oder Rd-dot. $Al_2O_3$ aus Epoxycyclododecan erhalten, wobei darauf hingewiesen wird, daß Zeolithe für diese Reaktion ungeeignet sind (Neftekhimya 16 (1976) 250-254)
In EP-PS 100 117 wird die Reaktion von Styroloxid und von am aromatischen Kern alkyl- bzw. alkoxylsubstituierte Styroloxiden an einem Titan-haltigen Zeolithen zu ß-Phenylaldehyden in der flüssigen Phase bei 30 bis 100 °C beschrieben. Der hierzu eingesetzte Katalysator wird aufwendig aus hochreinen Ausgangsstoffen wie Tetraalkylorthosilikat, Tetraalkylorthotitanat und Tetrapropylammoniumhydroxid hergestellt. Hohe Umsätze erzielt man nur bei Reaktion in einem Lösungsmittel wie Methanol oder Aceton bei 30 bis 100 °C in der Flüssigphase bei Verweilzeiten von 1 bis 1,5 h. Dies bedeutet erhöhten Aufwand für Destillation und Betrieb. An Titan-haltigen Zeolithen kann man nur Styroloxid und an Aromaten alkylierte bzw. alkoxylierte Styroloxide umsetzen.

Es war die Aufgabe gestellt, ein Verfahren zu entwickeln, wonach auch bisher teilweise nicht zugängliche Aldehyde bzw. Ketone aus den entsprechenden Epoxiden in Gegenwart von einfach verfügbaren Katalysatoren hoher Aktivität und leichter Regenerierbarkeit hergestellt werden können. Weiterhin sollten bei langen Katalysatorstandzeiten hohe Umsätze und Selektivitäten und eine flexible Einsetzbarkeit des Katalysators hinsichtlich der Edukte erreicht werden.

Gegenstand der Erfindung ist ein Verfahren zur Herstellung von Aldehyden und Ketonen der Formel (I)

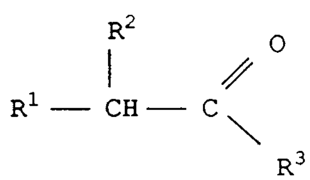

(I)

in der R1 und R2 gerade oder verzweigte Alkylreste mit einer C-Zahl von 1 bis 12, gerade oder verzweigte Alkenylreste mit einer C-Zahl von 1 bis 12, Cycloalkyl- oder Cycloalkenylreste mit 5 bis 8 Kohlenstoffatomen, Alkoxyreste oder Aryl- bzw. Aralkylreste, die ihrerseits substituiert sein können, bedeuten und $R^3$ ein Wasserstoff, Alkyl-, Alkenyl-, Aryl- oder Aralkylreste sein kann, dadurch gekennzeichnet, daß man Epoxide der Formel (II)

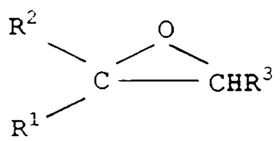

(II)

wobei $R^1$, $R^2$ und $R^3$ obige Bedeutung haben, unter Verwendung von einfach verfügbaren Katalysatoren hoher Aktivität und leichter Regenerierbarkeit umlagert.

Unter einfach verfügbaren Katalysatoren, hoher Aktivität und leichter Regenerierbarkeit im Sinne der Erfindung versteht man hydrothermal hergestellte Phosphate wie Aluminiumphosphate, Siliciumaluminiumphosphate, Eisenaluminiumphosphate oder Eisensiliciumaluminiumphosphate und Borphosphate sowie

Phosphorsäure und/oder Borsäure auf Bims, Siliciumdioxid bzw. Aluminiumoxid und Siliciumdioxid.

Durch die vorliegende Erfindung wird ein Verfahren zur Herstellung der Aldehyde und Ketone der Formel (I) aus gut zugänglichen Ausgangsstoffen der Formel (II) in die Technik eingeführt, bei dem in Gegenwart von Katalysatoren, die sich durch einfache Verfügbarkeit, hohe Aktivität und leichte Regenerierbarkeit auszeichnen, bei langen Katalysatorstandzeiten, hohen Umsätzen und hohen Selektivitäten ein flexibler Einsatz der Katalysatoren hinsichtlich der Endprodukte gewährleistet ist.

Das erfindungsgemäße Verfahren vermeidet die eingangs erwähnten Nachteile der bekannten Verfahren. Weitergehende Vorteile des erfindungsgemäßen Verfahrens sind: Durch vollständigen Umsatz und Selektivität > 90 % ergeben sich auch keine Trennprobleme. Bei langen Standzeiten werden auch sehr gute Ausbeuten erreicht. Vorteilhaft sind auch die einfache Isolierung der Endprodukte, in der Regel Weiterverwendung ohne zusätzliche Reinigung, leichte Regenerierbarkeit der Katalysatoren bei eventuell auftretender Verkokung. Ein weiterer Vorteil ist es, daß man die Reaktion in der Gasphase ausführen kann.

Für das erfindungsgemäße Verfahren können z.B. folgende Epoxide als Ausgangsstoffe verwendet werden: $\alpha$-Phenylstyroloxid (1,1-Diphenylethylenoxid), $\alpha$-Methylstyroloxid, $\alpha$-Ethylstyroloxid, $\alpha$-Isopropylstyroloxid, 2-Methyl-2,3-epoxibutan und Diisobutenoxid und andere.

Als Katalysatoren für die Herstellung von Aldehyden und Ketonen der Formel (I) aus entsprechenden Epoxiden der Formel (II) werden im folgenden beschrieben: Als Aluminiumphosphat-Katalysatoren werden für das erfindungsgemäße Verfahren unter hydrothermalen Bedingungen synthetisierte Aluminiumphosphate eingesetzt. Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP-PS 132 708, US-PS 4 310 440 und US-PS 4 473 663 beschrieben.

Beispielsweise $AlPO_4$-5 (APO-5) kann man synthetisieren, indem man Orthophosphorsäure mit Pseudoboehmit (Catapal SBI) in Wasser homogen mischt, zu dieser Mischung Tetrapropylammoniumhydroxyd gibt und danach bei etwa 150 °C 20 bis 60 h unter autogenem Druck in einem Autoklaven umsetzt. Das abfiltrierte $AlPO_4$-5 wird bei 100 bis 160 °C getrocknet und bei 450 bis 550 °C calciniert. $AlPO_4$-9 (APO-9) kann aus Orthophosphorsäure und Pseudoboehmit in wäßriger DABCO-Lösung (1,4-Diazabicyclo-(2,2,2)-octan) bei etwa 200 °C unter autogenem Druck während 200 bis 400 h synthetisiert werden. Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung ist in EP-PS 103 117 und US-PS 4 440 871 beschrieben. Man erhält diese Stoffe durch Kristallisation aus wäßriger Mischung bei 100 bis 250 °C unter autogenem Druck während 2 h bis 2 Wochen, wobei die Reaktionsmischung aus einer Silicium-, Aluminium- und Phosphorkomponente in wäßrigen aminoorganischen Lösungen umgesetzt wird. SAPO-5 beispielsweise wird durch Mischung von $SiO_2$, suspendiert in wäßriger Tetrapropylammoniunhydroxid-Lösung, mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C. Als Siliciumaluminiumphosphate sind z.B. auch ZYT-5, ZYT-6, ZYT-7, ZYT-9, ZYT-11 und ZYT-12 geeignet (J5 9217-619).

Borphosphate für das erfindungsgemäße Verfahren kann man durch Mischen und Kneten von konzentrierter Borsäure und Phosphorsäure und durch anschließende Trocknung und Calcination in Inertgas-, Luft- oder Dampfatmosphäre bei 250 bis 650 °C, vorzugsweise 300 bis 500 °C, herstellen. Geeignet für das erfindungsgemäße Verfahren ist auch $CePO_4$. $CePO_4$ wird z.B. durch Fällung aus 52 g $(Ce(NO_3)_3 \times 6H_2O$ und 56 g $NaH_2PO_4 \times 2H_2O$ erhalten. Nach der Filtration wird das Material zu Strängen verformt, bei 120 °C getrocknet und bei 450 °C calciniert. $CePO_4$ enthält 47,1 Gew.-% Ce und 12,7 Gew.% P. Geeignete Phosphate sind z.B. auch $FePO_4$ und $Zr_3(PO_4)_4$.

Für die Katalyse der erfindungsgemäßen Reaktion kann man auch Borsäure auf $SiO_2$-, $Al_2O_3$- oder Bims-Trägern z.B. durch Auftränken oder Versprühen aufgebracht einsetzen. Auch Siliciumdioxid kann als Katalysator eingesetzt werden.

Die Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Die erfindungsgemäße Umwandlung der Epoxide wird bevorzugt in der Gasphase und in der Regel bei Temperaturen von 50 bis 500 °C, vorzugsweise 150 bis 400 °C, und einer Belastung WHSV = 0,1 bis 20 $h^{-1}$, vorzugsweise 0,5 bis 5 $h^{-1}$ (g Epoxid/g Katalysator und Stunde) ausgeführt. Die Reaktion kann im Festbett oder im Wirbelbett ausgeführt werden. Im allgemeinen steigt der Umsatz mit steigender Temperatur stark an, während die Selektivität in einem bestimmten Temperaturbereich nur wenig zurückgeht.

Es ist auch möglich, die Reaktion in der Flüssigphase (Suspensions-, Riesel- oder Sumpffahrweise) durchzuführen.

Das Verfahren wird im allgemeinen bei Normaldruck oder bei vermindertem oder erhöhtem Druck diskontinuierlich, vorzugsweise kontinuierlich durchgeführt.

Schwerflüchtige oder feste Ausgangsstoffe werden in gelöster Form z.B. in THF-, Toluol- oder Petrolether-Lösung eingesetzt. Allgemein ist auch eine Verdünnung mit diesen Lösungsmitteln oder Inertgasen wie $N_2$, Ar, $H_2O$-Dampf möglich.

Nach der Umsetzung kann man die entstandenen Aldehyde und Ketone nach üblichen Techniken, z.B. durch Destillation aus dem Reaktionsgemisch isolieren; nicht-umgesetzte Ausgangsstoffe werden gegebenenfalls, wenn nötig, in die Umsetzung zurückgeführt. Auch eine direkte Weiterverarbeitung der Reaktionsprodukte ist aufgrund der sehr hohen Ausbeuten möglich. Bei dem erfindungsgemäßen Verfahren fallen vorzugsweise die monomeren Verbindungen an. Wenn auch Oligomere, z.B. trimere Aldehyde gebildet werden, so kann man diese abtrennen und nach bekannten Methoden zu den gewünschten Monomeren spalten.

Durch Wahl des Katalysators und der Reaktionsbedingungen, insbesondere der Temperatur, kann bei der Umsetzung der Epoxide der Formel (II) das Verhältnis Aldehyd zu Keton beeinflußt werden. Aldehyde lagern sich teilweise in Ketone um, wie in DE-OS 34 19 378 beschrieben, bzw. dehydratisieren zu Dienen wie in DE-OS 34 19 379 beschrieben.

Die nach dem erfindungsgemäßen Verfahren zugänglichen teilweise neuen Verbindungen sind wichtige Zwischenprodukte und können nach geläufigen Methoden auf einfachem Wege zu Aminen, Alkoholen und Säuren, z.B. durch Oxidation mit Sauerstoff oder durch Reduktion z.B. durch katalytische Hydrierung oder aminierende Hydrierung weiterverarbeitet werden.

Beispiele 1 bis 10

Die Reaktionen werden unter isothermen Bedingungen in einem Rohrreaktor (Wendel 0,6 cm Durchmesser, 90 cm Länge) in der Casphase mindestens 6 Stunden lang durchgeführt. Die Trennung und Charakterisierung der Reaktionsprodukte erfolgt nach üblichen Methoden. Die quantitative Bestimmung der Reaktions produkte und der Ausgangsstoffe erfolgt gaschromatographisch und durch CO-Zahl.

Die in den Beispielen verwendeten Katalysatoren sind:

Katalysator G

$AlPO_4$-12 (APO-12) wird synthetisiert wie APO-9 (Katalysator L), wobei anstelle von 112 g DABCO 60 g Ethylendiamin eingesetzt werden. Die Reaktionsbedingungen sind 200 °C für 24 Stunden. Das so synthetisierte Material enthält nach Trocknung bei 120 °C und Calcination 500 °C/16 h 55,5 Gew.-% $P_2O_5$ und 39,7 Gew.-% $Al_2O_3$. Der APO-12 wird wie APO-9 verformt.

Katalysator H

Die Synthese von $AlPO_4$-21 (APO-21) erfolgt durch Zusammenrühren von 200 g 98 %ige Phosphorsäure, 156 g gefällten Aluminiumhydroxid und 71 g Pyrrolidon in 900 g Wasser und anschließende Reaktion bei 200 °C unter autogenem Druck während 91 h. Das bei 120 °C getrocknete und bei 500 °C calcinierte Produkt enthält 56,5 Gew.% $P_2O_5$ und 43,4 Gew.-% $Al_2O_3$. Dieses $AlPO_4$-21 wird mit einem Verstrangungshilfsmittel zu 2 mm-Strängen verformt, bei 110 °C getrocknet und bei 500 °C/16 h calciniert.

Katalysator I

Siliciumaluminiumphosphat-5 (SAPO-5) wird hergestellt aus einer Mischung aus 200 g 98 %ige Phosphorsäure, 136 g Boehmit, 60 g Kieselsol (30 %ig), 287 g Tripropylamin und 587 g $H_2O$. Diese Mischung wird bei 150 °C während 168 h unter autogenem Druck umgesetzt. Nach Filtration wird das kristalline Produkt bei 120 °C getrocknet und bei 500 °C calciniert. SAPO-5 enthält 49,8 Gew.-% $P_2O_5$, 33,0 Gew.% $Al_2O_3$, 6,2 Gew.% $SiO_2$. SAPO-5 wird mit einem Verstrangungshilfsmittel zu 3 mm Strängen verformt, bei 120 °C getrocknet und bei 500 °C calciniert.

Katalysator J

Siliciumaluminiumphosphat-11 (SAPO-11) wird synthetisiert aus einer Mischung von 200 g $H_3PO_4$, 136

g Boehmit, 60 g Kieselsol (30 %ig), 91 g Dipropylamin und 890 g Wasser. Die Umsetzung wird bei 200 °C während 96 h unter autogenem Druck durchgeführt. Nach Filtrieren wird bei 120 °C getrocknet und bei 500 °C calciniert. SAPO-11 enthält 47,7 Gew.-% $P_2O_5$,39,4 Gew.-% $Al_2O_3$ und 6,4 Gew.-% $SiO_2$. Dieses kristalline Produkt wird mit einem Verstrangungshilfsmittel zu 3 mm Strängen verformt, bei 120 °C und bei 500 °C/16 h calciniert.

Katalysator K

BPO₄ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3 mm Strängen verformt. Diese Stränge werden bei 100 °C getrocknet und bei 350 °C calciniert. Katalysator K enthält 8,77 Gew.-% B und 28,3 Gew.-% P.

Katalysator L

$AlPO_4$-9 (APO-9) wird synthetisiert, indem man 200 g 98 %ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200 °C während 336 h unter autogenem Druck umsetzt. Nach Abfiltrieren wird das kristalline Material bei 120 °C getrocknet und bei 500 °C/16 h calciniert. Das so synthetisierte $AlPO_4$-9 enthält 49,0 Gew.-% $P_2O_5$, 37,1 Gew.-% $Al_2O_3$. Dieses Material wird mit einem Verstrangungshilfsmittel zu 3 mm Strängen verformt, abermals bei 120 °C getrocknet und bei 500 °C/6 h calciniert.

Katalysator M

$SiO_2$ im Handel erhältlich als D11-11®.

Katalysator N

KC-Trockenperlen WS® mit etwa 97 % $SiO_2$ und etwa 3 % $Al_2O_3$ werden mit $H_3BO_3$ gelöst in $CH_3OH$ getränkt, bei 110 °C getrocknet und bei 500 °C/5 h calciniert. Der Borgehalt beträgt 15,7 Gew.-% ($B_2O_3$).

Die Versuchsergebnisse sind in den folgenden Tabellen zusammengestellt.

Tabelle 1

| Beispiel | 1 | 2 | 3 | 4 | 5 |
|---|---|---|---|---|---|
| Einsatzstoff | 2-Methylstyroloxid | | | | |
| Katalysator | K | L | G | M | J |
| Temperatur | 300°C | 300°C | 300°C | 300°C | 300°C |
| WHSV | 2,3 $h^{-1}$ | 2,3 $h^{-1}$ | 2,3 $h^{-1}$ | 2,5 $h^{-1}$ | 2,5 $h^{-1}$ |
| Umsatz % | 100 | 100 | 100 | 100 | 100 |
| Wertprodukte im Austrag in Gew-% | | | | | |
| 2-Phenylpropanal | 82,4 | 87,8 | 47,7 | 92,4 | 72,6 |
| Phenylaceton | 11,3 | 0,9 | 28,8 | 1,2 | 3,1 |

Tabelle 2

| Beispiel | 6 |
|---|---|
| Einsatzstoff | 1,1-Diphenylethylenoxid[1)] |
| Katalysator | K |
| Temperatur | 300°C |
| WHSV | 2,1 $h^{-1}$ |
| Umsatz % | 100 |
| Wertprodukte im Austrag in Gew-%[2)] | |
| Diphenylacetaldehyd | 93,3 |
| Phenylbenzylketon | 1,9 |

1) 1,1-Diphenylethylenoxid in THF im Gewichtsverhältnis 50 : 50 gelöst

2) THF herausgerechnet

Tabelle 3

| Beispiel | 7[2] | 8[2] | 9[2] | 10[2] |
|---|---|---|---|---|
| Einsatzstoff | Diisobutylenoxid[1] | | | |
| Katalysator | L | J | N | M |
| Temperatur | 300°C | 300°C | 300°C | 300°C |
| WHSV | 1,8 $h^{-1}$ | 1,6 $h^{-1}$ | 2,3 $h^{-1}$ | 2,0 $h^{-1}$ |
| Umsatz % | 100 | 100 | 100 | 100 |
| Wertprodukte im Austrag in Gew-% | | | | |
| 2,2,4-Trimethylpentanal | 69,8 | 70,8 | 63,6 | 69,9 |

[1] Das eingesetzte Diisobutylenoxidgemisch enthält 70,9 Gew.-% 2,2,4-Trimethyl-4,5-epoxipentan und 20,9 Gew.-% 2,2,4-Trimethyl-3,4-epoxipentan

[2] Diisobutylenoxidgemisch mit THF im Gewichtsverhältnis 50 : 50 verdünnt.

## Patentansprüche

1. Verfahren zur Herstellung von Aldehyden und Ketonen der Formel (I)

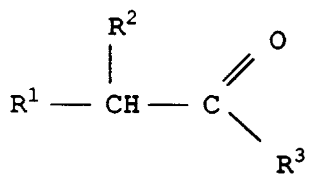

(I)

in der $R^1$ und $R^2$ gerade oder verzweigte Alkylreste mit einer C-Zahl von 1 bis 12, Alkenylreste mit einer C-Zahl von 1 bis 12, Cycloalkyl- oder Cycloalkenylreste mit 5 bis 8 Kohlenstoffatomen, Alkoxyreste

oder Aryl- bzw. Aralkylreste, die ihrerseits substituiert sein können, bedeuten und $R^3$ Wasserstoff, Alkyl-, Alkenyl-, Aryl- oder Aralkylreste sein kann, dadurch gekennzeichnet, daß man Epoxide der Formel (II)

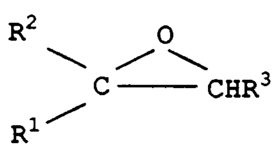

(II)

in der $R^1$, $R^2$ und $R^3$ obige Bedeutung haben, an hydrothermal hergestellten Phosphaten und/oder Phosphaten des B, Fe, Zr, Ce katalytisch umlagert.

2. Verfahren nach Anspruch 1, dadurch gekennzeichnet, daß hydrothermal hergestelltes Aluminiumphosphat und/oder Siliciumaluminiumphosphatund/oder Eisenaluminiumphosphat und/oder Eisensiliciumaluminiumphosphat eingesetzt werden.

3. Verfahren zur Herstellung von Aldehyden und Ketonen der Formel (I)

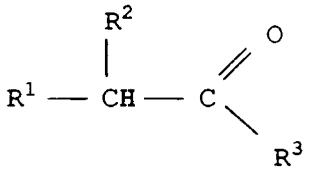

(I)

in der $R^1$ und $R^2$ gerade oder verzweigte Alkylreste mit einer C-Zahl von 1 bis 12, Alkenylreste mit einer C-Zahl von 1 bis 12, Cycloalkyl- oder Cycloalkenylreste mit 5 bis 8 Kohlenstoffatomen, Alkoxyreste oder Aryl- bzw. Aralkylreste, die ihrerseits substituiert sein können, bedeuten und $R^3$ Wasserstoff, Alkyl-, Alkenyl-, Aryl- oder Aralkylreste sein kann, dadurch gekennzeichnet, daß man Epoxide der Formel (II)

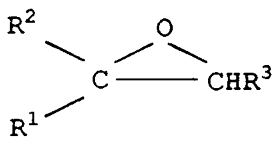

(II)

in der $R^1$, $R^2$ und $R^3$ obige Bedeutung haben, an Borsäure auf Bims bzw. Siliciumdioxid bzw. Aluminiumoxid katalytisch oder an Siliciumdioxid umlagert.

4. Verfahren nach Anspruch 1 bis 3, dadurch gekennzeichnet, daß als Verbindungen der Formel II α-Methylstyroloxid, Diisobutylenoxid, 2-Methyl-2,3-epoxibutan und 1,1-Diphenylethylenoxid eingesetzt werden.